# FASCICULE DE BREVET EUROPEEN

(11) **EP 0 932 824 B1**
(45) Date de publication et mention de la délivrance du brevet: **10.11.2004**
(21) Numéro de dépôt: 98941534.4
(22) Date de dépôt: 30.07.1998
(51) Int. Cl.: G01N 1/00, G01N 35/10, B01L 3/00, A61M 5/32, A61M 5/162

(54) **DISPOSITIF POUR LE PRELEVEMENT ET/OU L'INJECTION A L'INTERIEUR D'UN TUBE D'ECHANTILLON BOUCHE**
GERÄT ZUR ENTNAHME AUS DEM INNEREN UND/ODER INJEKTION IN DAS INNERE EINES VERKORKTEN PROBENGEFÄSSES
DEVICE FOR SAMPLING AND/OR INJECTING INSIDE A PLUGGED SAMPLE TUBE

(30) Priorité: 20.08.1997 FR 9710566
(43) Date de publication de la demande: 04.08.1999
(73) Titulaire: Stago Instruments, 92230 Gennevilliers (FR)
(72) Inventeur: FERE, Patrick, F-75004 Paris (FR); PERIN, Patrick, F-78210 Saint Cyr l'Ecole (FR); ROUSSEAU, Alain, F-75004 Paris (FR)
(74) Mandataire: de Saint-Palais, Arnaud Marie
(86) Numéro de dépôt international: PCT/FR1998/001734
(87) Numéro de publication internationale: WO 1999/009389

(56) Documents cités:
- WO-A-98/36259
- DE-A- 3 901 068
- DE-A- 19 512 607
- DE-U- 29 807 150
- US-A- 4 585 446
- US-A- 5 163 582
- US-A- 5 220 947
- US-A- 5 413 000

## Description

La présente invention concerne un dispositif destiné à effectuer des prélèvements et/ou des injections à l'intérieur de récipients fermés tels que, par exemple, des tubes d'échantillons refermés par des bouchons en matière plastique.

D'une manière générale, on sait que pour des raisons de sécurité et de précision, dans de nombreux processus d'analyse, en particulier des analyses biologiques, on utilise des tubes d'échantillons bouchés à l'aide de bouchons en caoutchouc ou plus généralement en élastomère, de manière à pouvoir exécuter toutes les manipulations rentrant dans le cadre de ces processus sans avoir à ôter le bouchon et donc sans avoir un accès direct à l'échantillon. Dans le cas d'analyses hématologiques, on emploie de plus en plus fréquemment des tubes d'échantillons fermés dans lesquels est créé un vide relatif permettant lors de la prise de sang, d'éviter l'emploi d'une seringue : le sang est directement aspiré dans le tube sous l'effet de la dépression.

Les opérations de prélèvement et/ou d'injection effectuées dans ces tubes impliquent alors l'usage de dispositifs comprenant chacun une aiguille creuse raccordée à un conduit d'aspiration et/ou de refoulement et spécialement conçue pour pouvoir percer les bouchons en vue de pénétrer à l'intérieur du tube. Dans le cas où il équipe un automate d'analyse, le dispositif de prélèvement est porté par une tête de pipetage équipée de moyens permettant d'assurer les déplacements verticaux de l'aiguille, et éventuellement mobile au-dessus d'une aire de pipetage dans laquelle se trouve disposée une pluralité de tubes d'échantillons. Il est clair que dans ce cas, l'aiguille devra pouvoir effectuer une multiplicité de percements et de prélèvements sans que la précision du dispositif en soit affectée.

On constate en pratique que la mise au point d'un tel dispositif et la conception de telles aiguilles de prélèvement posent de nombreux problèmes.

Ainsi, l'usage d'aiguilles creuses classiques à pointes effilées sensiblement coniques ne conviennent pas bien dans des appareils d'analyse automatiques ou semi-automatiques pour les raisons suivantes :
- Lors du percement du bouchon, ces aiguilles se comportent comme des emporte-pièce, en découpant, dans le bouchon, une pièce circulaire de section sensiblement égale à la section du canal de l'aiguille Cette pièce circulaire s'engage dans le canal en provoquant au moins partiellement son obturation. De ce fait, lors de la phase d'aspiration, cette pièce engendre une perte de charge plus ou moins importante qui constitue une première source d'erreur sur les quantités prélevées (lesquelles doivent demeurer constantes à chaque prélèvement).
- Au cours de l'action de percement, le bouchon subit une déformation engendrant une variation de pression (surpression) à l'intérieur du récipient, variation qui constitue une seconde source d'erreur sur les quantités prélevées. De même, lors de l'extraction de l'aiguille, la déformation du bouchon en sens inverse engendre une dépression provoquant un effet de succion qui tend à aspirer le liquide prélevé. L'imprécision résultant de ces deux phénomènes est amplifiée par le fait que le niveau d'échantillon à l'intérieur du tube est variable d'un tube à l'autre et, qu'en conséquence, les valeurs de la surpression et de la dépression ne peuvent donc pas être prédéterminées.
- L'effet d'emporte-pièce de l'aiguille conduit à la formation de perçages qui se referment mal après l'extraction de l'aiguille de sorte que l'étanchéité du bouchage ne peut plus être garantie.

Pour résoudre les problèmes relatifs à l'effet emporte-pièce, on a déjà proposé, par les brevets DE195 12 607 et US 5 220 947, d'utiliser des aiguilles dont les canaux sont bouchés au niveau de leurs extrémités pointues et qui présentent des perçages latéraux réalisés dans les corps des aiguilles. Ces aiguilles s'avèrent coûteuses et difficilement réalisables industriellement, à des cadences de production élevées.

Pour résoudre les problèmes relatifs à l'imprécision des quantités prélevées, on a proposé d'utiliser des dispositifs de prélèvement utilisant des jeux d'électrovannes permettant d'effectuer des séquences de prélèvement complexes tenant compte des paramètres précédemment évoqués. Toutefois, cette solution s'avère coûteuse, peu fiable et ne résout pas cependant tous les problèmes précédemment évoqués.

L'invention a donc plus particulièrement pour but d'apporter une solution simple, peu coûteuse et cependant efficace à ces problèmes.

Elle propose, à cet effet, un dispositif de prélèvement mettant en oeuvre une aiguille creuse mobile axialement de manière à pouvoir perforer un bouchon refermant le récipient dans lequel se trouve contenu l'échantillon à prélever.

Ce dispositif met en oeuvre une aiguille creuse mobile axialement de manière à pouvoir perforer le bouchon, l'extrémité pointue de l'aiguille comprenant au moins une arête tranchante tangente à une génératrice du corps cylindrique de l'aiguille, une forme oblique reliant l'arête audit corps cylindrique et un orifice latéral de sortie du canal centré sur ladite génératrice en un emplacement distant de l'arête, cet orifice étant axé perpendiculairement à l'axe longitudinal de l'aiguille.

Selon l'invention, ce dispositif est caractérisé en ce ladite extrémité du corps de l'aiguille est coudée et usinée de manière à comprendre une face d'usinage tangente à ladite génératrice, perpendiculaire au plan de symétrie de ladite extrémité coudée et dans laquelle débouche le canal de l'aiguille par ledit orifice, l'extrémité de cette face d'usinage constituant l'amorce de l'arête tranchante.

Avantageusement, la susdite arête et le susdit orifice s'étendent dans un même plan tangent à ladite génératrice.

Selon un mode d'exécution préféré de l'invention, la susdite forme oblique constitue un coudage de l'extrémité du corps cylindrique tandis que le susdit plan tangent est un plan d'usinage perpendiculaire au plan de symétrie du coudage.

Selon une autre particularité de l'invention, le corps cylindrique de l'aiguille comprend au niveau de sa surface cylindrique extérieure au moins une gorge axiale se terminant à une distance prédéterminée de la pointe de l'aiguille. De préférence, cette gorge, qui est destinée à assurer une mise à l'atmosphère du volume intérieur de l'échantillon pendant le prélèvement, présente une section carrée ou rectangulaire.

Un mode d'exécution de l'invention sera décrit ci-après, à titre d'exemple non limitatif, avec référence aux dessins annexés dans lesquels :
La figure 1 est une représentation schématique d'un dispositif de prélèvement classique ;
Les figures 2 et 3 sont des vues partielles illustrant les déformations du tube d'échantillon lors du perçage du bouchon (figure 2) et lors de l'extraction de l'aiguille (figure 3) ;
La figure 4 est une vue de côté de l'aiguille utilisée conformément à l'invention, dans un plan parallèle au plan longitudinal de symétrie de l'aiguille ;
La figure 5 est une vue de côté de l'aiguille à 90° de la vue de la figure 4 ;
La figure 6 est une coupe axiale de l'extrémité de l'aiguille ;
Les figures 7 et 8 sont des coupes respectivement selon A/A' et selon B/B' de la figure 6 ;
La figure 9 est une représentation schématique permettant d'illustrer le principe du percement du bouchon grâce à l'aiguille représentée figures 4 à 8.

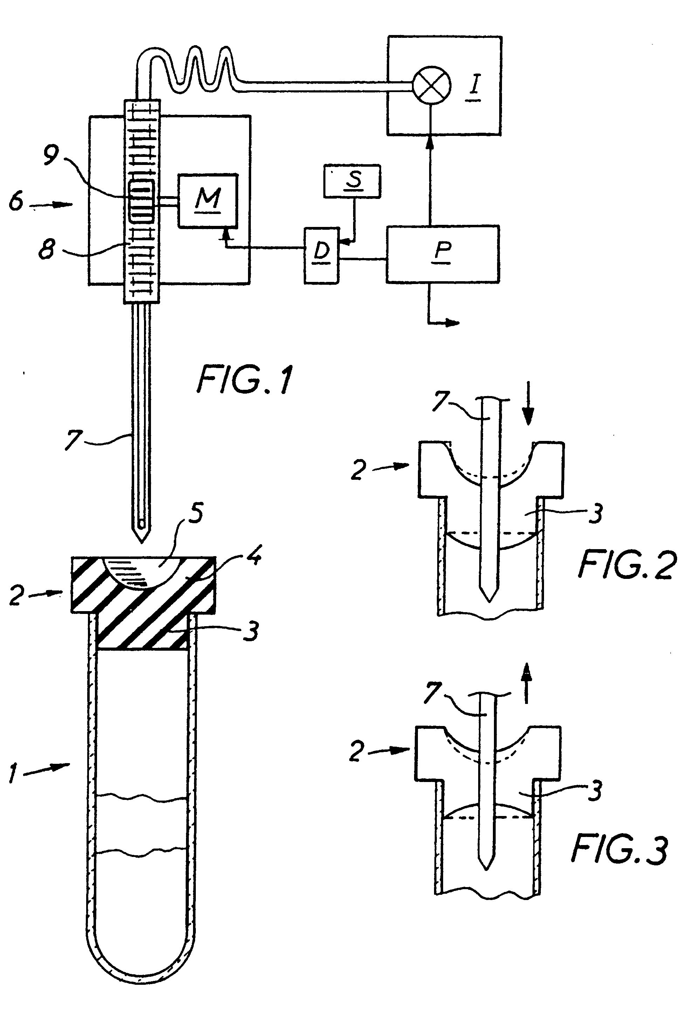

Dans l'exemple représenté sur la figure 1, le récipient contenant l'échantillon à analyser consiste en un tube de verre de type classique 1 refermé par un bouchon en caoutchouc 2 comprenant un corps cylindrique 3 de diamètre légèrement supérieur au diamètre intérieur du tube 1 et un chapeau 4, également cylindrique de diamètre supérieur au diamètre extérieur du tube 1. La face supérieure du chapeau 4 comprend un évidement sphérique 5 destiné à réduire l'épaisseur de la région centrale où s'effectuent les perçages. Bien entendu, le corps 3 s'engage avec étanchéité dans le tube 1 tandis que le chapeau 4 forme avec le corps 3 un épaulement sur lequel le bord supérieur du tube 1 vient en butée.

Dans l'axe du tube 1 est représentée une tête de pipetage 6 mobile perpendiculairement audit axe et qui comprend un mécanisme d'actionnement d'une aiguille 7 axée parallèlement audit axe.

Ce mécanisme comprend une crémaillère 8 solidaire de l'aiguille et sur laquelle engrène un pignon 9 entraîné par un moteur électrique à courant continu M alimenté par une source de courant électrique S dont les caractéristiques d'intensité et/ou de tension sont mesurées par un détecteur D avant d'être transmises à un processeur P qui assure la commande du dispositif. L'aiguille 7 est, quant à elle, connectée à un circuit de prélèvement/injection I également piloté par le microprocesseur P.

Le fonctionnement de ce dispositif est alors le suivant : Lors d'une phase de prélèvement et/ou d'injection, la tête de pipetage 6 se dispose au-dessus du tube 1, l'aiguille 7 étant située dans l'axe longitudinal du tube 1.

Le processeur P commande alors la rotation du moteur M de manière à provoquer un déplacement de la crémaillère 8 et donc de l'aiguille 7 à laquelle elle se trouve fixée.

L'aiguille 7 subit donc un déplacement en translation jusqu'à ce qu'elle atteigne le bouchon 2. Au contact de la pointe de l'aiguille sur le bouchon 2, le couple résistant qui en résulte au niveau du moteur M modifie les caractéristiques du courant d'alimentation. Ces modifications sont détectées par le détecteur D qui transmet une information correspondante au processeur P. Au cours de la perforation du bouchon 2, l'aiguille 7 exerce sur le corps en caoutchouc 3 une contrainte engendrant une déformation vers le bas (bombement) dudit corps et, parallèlement, un accroissement du volume de la cavité (figure 2). Cette déformation provoque donc un accroissement de pression à l'intérieur du tube 1. Même dans le cas où cette surpression se trouve compensée par une circulation de gaz dans l'aiguille 7, la pression à l'intérieur du tube 1 demeure indéterminée. De ce fait, la quantité prélevée sera imprécise.

Lors de l'extraction de l'aiguille 7, le corps 3 du bouchon 2 se trouve soumis à une contrainte inverse de la précédente : La face inférieure du corps du bouchon 2 se déforme pour prendre une forme concave en engendrant une dépression à l'intérieur du tube (figure 3). Cette dépression va créer une succion du liquide prélevé par l'aiguille de sorte qu'on ajoute une nouvelle imprécision à la quantité d'échantillon prélevée. Ce double phénomène varie en outre en fonction du niveau de l'échantillon à l'intérieur du tube 1.

Bien entendu, à ces causes d'imprécision s'ajoute celle relative au fait qu'une aiguille classique se comporte comme un emporte-pièce et découpe une pièce de caoutchouc susceptible d'obturer au moins partiellement l'aiguille 7.

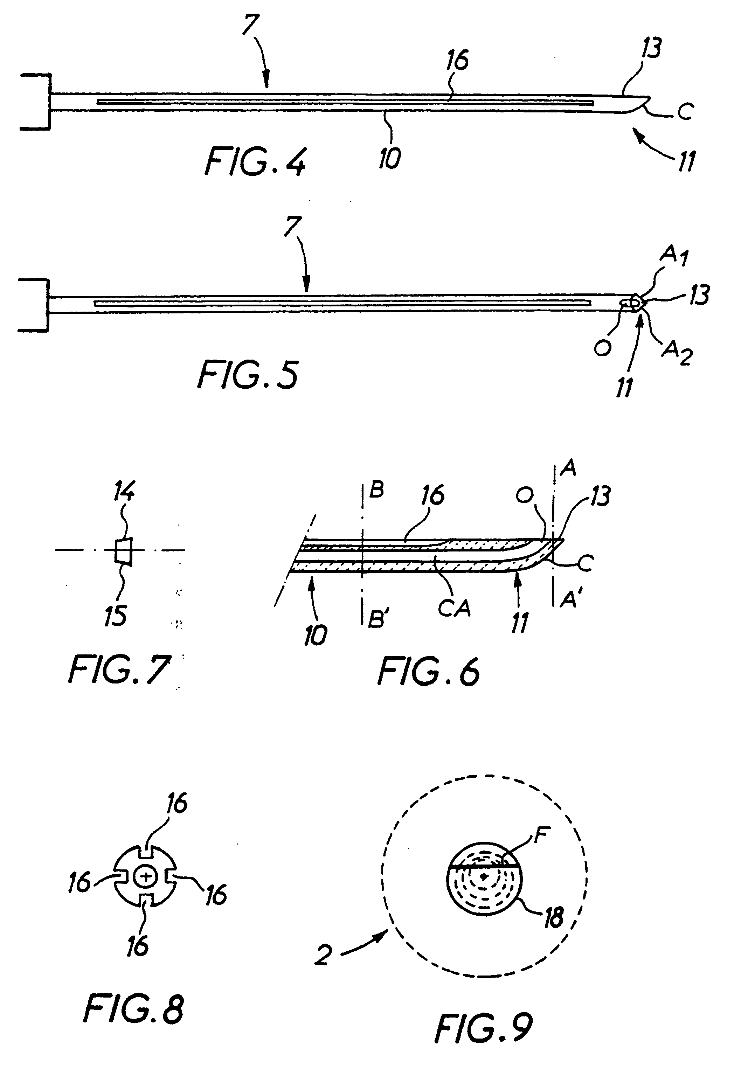

Telle qu'illustrée sur les figures 4 à 8, l'aiguille selon l'invention permet d'éviter ces inconvénients.

Cette aiguille 7 comprend un corps creux cylindrique 10 dont l'extrémité pointue 11 est obtenue par coudage de l'extrémité du corps 10 puis par usinage de l'extrémité coudée de manière à réaliser une face 13 tangente à une génératrice du corps 10 et perpendiculaire au plan de symétrie de ladite partie coudée C.

On obtient donc une face d'usinage 13 plane dont l'extrémité ovoïde constitue l'amorce de l'arête tranchante. Cette arête tranchante A₁, A₂ est affûtée par meulage de deux facettes latérales 14, 15 qui lui confèrent une forme pointue en V visible sur la figure 5.

Par ailleurs, la surface extérieure du corps 7 comprend quatre gorges longitudinales 16 diamétralement opposées deux à deux et qui présentent chacune une section carrée.

Ces gorges longitudinales 16 qui sont réalisées sur une grande partie de la longueur de l'aiguille 7 se terminent à une distance prédéterminée de la pointe 11 de manière à n'être jamais au contact du liquide que l'on désire prélever.

Grâce à ces dispositions, lors de la perforation du bouchon 2, les arêtes tranchantes A₁, A₂ de la pointe 11 effectuent une coupure rectiligne du caoutchouc en évitant ainsi tout phénomène d'emporte-pièce. Cette fente F est ensuite élargie élastiquement d'un côté par la partie coudée jusqu'à ce qu'elle atteigne le corps 10 en prenant progressivement une forme circulaire 18. Au cours de cette perforation, la face d'usinage 13 dans laquelle débouche l'orifice O de sortie du canal CA de l'aiguille glisse sur la face de la fente F sur laquelle s'exerce la plus faible contrainte et qui n'est que très faiblement déformée (elle reste plane). De ce fait, le caoutchouc ne pénètre pas dans l'orifice O et l'on ne court aucun risque de bouchage extérieur.

Une fois que la pointe de l'aiguille 7 a traversé le bouchon 2 et que les gorges 16 ressortent à l'intérieur du tube 1, on obtient une mise à l'atmosphère du volume intérieur du tube 1. La pression qui règne dans ce dernier au moment de la phase de prélèvement est donc ramenée à une valeur constante (pression atmosphérique). Les quantités d'échantillon prélevées ne sont donc plus sujettes aux variations de pression évoquées et sont donc relativement précises.

Lors de l'extraction de l'aiguille 7, l'extrémité de l'aiguille qui a été au contact de l'échantillon se trouve essuyée d'autant mieux qu'il n'y a pas eu d'enlèvement de caoutchouc et que la fente se referme progressivement au fur et à mesure que la section de l'aiguille diminue.

Lorsque l'aiguille est extraite, la fente est complètement refermée et la précontrainte exercée par le tube sur le corps garantit une parfaite étanchéité.

## Revendications

1. Dispositif pour le prélèvement et/ou l'injection de liquide à l'intérieur d'un récipient (1) fermé par un bouchon (2), ce dispositif mettant en oeuvre une aiguille creuse (7) mobile axialement de manière à pouvoir perforer le bouchon (2), l'extrémité pointue (11) de l'aiguille (7) comprenant au moins une arête tranchante (A₁, A₂) tangente à une génératrice du corps cylindrique (10) de l'aiguille (7), une forme oblique (C) reliant l'arête (A₁, A₂) audit corps cylindrique (10) et un orifice latéral (0) de sortie du canal (CA) centré sur ladite génératrice en un emplacement distant de l'arête (A₁, A₂), cet orifice (O) étant axé perpendiculairement à l'axe longitudinal de l'aiguille (7), **caractérisé en ce que**,
ladite extrémité du corps de l'aiguille est coudée et usinée de manière à comprendre une face d'usinage (13) tangente à ladite génératrice, perpendiculaire au plan de symétrie de ladite extrémité coudée et dans laquelle débouche le canal (CA) de l'aiguille (7) par ledit orifice (O), l'extrémité de cette face d'usinage (13) constituant l'amorce de l'arête tranchante (A₁, A₂).

2. Dispositif selon la revendication 1,
**caractérisé en ce que** le susdit orifice (O) et la susdite arête (A₁, A₂) s'étendent dans un même plan tangent à ladite génératrice.

3. Dispositif selon la revendication 1,
**caractérisé en ce que** le corps cylindrique (10) de l'aiguille (7) comprend au niveau de sa surface cylindrique extérieure au moins une gorge axiale (16) se terminant à une distance prédéterminée de la pointe (11) de l'aiguille.

4. Dispositif selon la revendication 3,
**caractérisé en ce que** la susdite gorge (16) présente une section carrée ou rectangulaire.

5. Dispositif selon l'une des revendications 3 et 4,
**caractérisé en ce que** le susdit corps (10) comprend quatre gorges longitudinales (16) diamétralement opposées deux à deux.

6. Dispositif selon l'une des revendications précédentes,
**caractérisé en ce que** l'arête tranchante (A₁, A₂) présente une forme pointue grâce au meulage de deux facettes latérales (14, 15).

7. Dispositif selon l'une des revendications précédentes,
**caractérisé en ce que** le déplacement en translation de l'aiguille (7) est commandé par un moteur électrique (M) alimenté par un circuit incluant un détecteur (D) apte à détecter un paramètre représentatif du couple résistant engendré lors du contact de la pointe de l'aiguille (7) sur le bouchon (2).

## Patentansprüche

1. Vorrichtung für die Flüssigkeitsentnahme und/oder -einspritzung in einen mit einem Stopfen (2) verschlossenen Behälter (1), die eine axial bewegliche hohle Nadel (7) so in Bewegung setzt, um den Stopfen (2) durchstechen zu können, wobei das zugespitzte Ende (11) der Nadel (7) mit mindestens einer die Mantellinie des zylindrischen Körper (10) der Nadel (7) berührenden Scharfkante (A₁, A₂) versehen ist, einer Schrägform (C), die die Kante (A₁, A₂) mit dem genannten zylindrischen Körper (10) verbindet und eine seitliche Öffnung (O) des Kanalabgangs (CA), die auf besagter Mantellinie auf einen von der Kante (A₁, A₂) entfernten Standort zentriert ist, wobei diese Öffnung (O) im rechten Winkel zur Längsachse der Nadel (7) liegt, **dadurch gekennzeichnet, dass** besagtes Körperende der Nadel gebogen und so bearbeitet ist, dass es eine tangentiale Bearbeitungsseite (13) zur besagten Mantellinie umfasst, indem rechtwinkelig zur Symmetrieebene des genannten gebogenen Endes, in welchem der Kanal (CA) der Nadel (7) durch besagte Öffnung (O) mündet, das Ende dieser Bearbeitungsseite (13) den Anriss der Scharfkante (A₁, A₂) bildet.

2. Vorrichtung nach Anspruch 1, **dadurch gekennzeichnet, dass** die besagte Öffnung (0) und die besagte Kante (A₁, A₂) sich über dieselbe Tangentialebene zur besagten Mantellinie hin erstrecken.

3. Vorrichtung nach Anspruch 1, **dadurch gekennzeichnet, dass** der zylindrische Körper (10) der Nadel (7) auf der Höhe ihrer äußeren zylindrischen Oberfläche mindestens eine axiale Rille (16) umfasst, die auf einer vorgegebenen Entfernung von der Spitze (11) der Nadel endet.

4. Vorrichtung nach Anspruch 3, **dadurch gekennzeichnet, dass** die besagte Rille (16) einen viereckigen oder rechteckigen Abschnitt aufweist.

5. Vorrichtung nach einem der Ansprüche 3 und 4, **dadurch gekennzeichnet, dass** der besagte Körper (10) vier diametral, d.h. zwei zu zwei, gegenüberliegende Längsrillen (16) umfasst.

6. Vorrichtung nach einem der vorherigen Ansprüche, **dadurch gekennzeichnet, dass** die Scharfkante (A₁, A₂) eine zugespitzte Form dank der Riefung von zwei Seiten-Facetten (14, 15) aufweist.

7. Vorrichtung gemäß einer der vorherigen Ansprüche, **dadurch gekennzeichnet, dass** die Verschiebung der Nadel (7) mit einem Elektromotor (M) gesteuert wird, der von einer Schaltung gespeist wird, die einen geeigneten Sensor (D) umfasst, der dazu fähig ist, einen repräsentativen Parameter für den Widerstandsmoment nach der Kontaktierung der Nadelspitze (7) auf dem Stopfen (2) zu erfassen.

## Claims

1. Device for the sampling and/or injecting of liquid into a receptacle (1) sealed by a stopper (2), this device using a hollow needle (7) that is axially mobile so as to be capable of perforating the stopper (12), the pointed tip (11) of the needle (7) comprising at least one cutting edge (A₁, A₂) at a tangent to a generating line of the cylindrical body (10) of the needle (7), an oblique shape (C) connecting the edge (A₁, A₂) to said cylindrical body (10) and a lateral outlet hole (O) of the channel (CA) centered on said generating line at a location distant from the edge (A₁, A₂), this hole (O) being angled perpendicularly to the longitudinal axis of the needle (7) **characterized in that**
the said tip of the body of the needle is cranked and machined so as to comprise a machined face (3) at a tangent to said generating line, perpendicular to the plane of symmetry to said cranked tip and into which the channel (CA) of the needle (7) opens by the said hole (O), the extremity of this machined face (13) constituting the start of the cutting edge (A₁, A₂).

2. Device according to claim 1,
**characterized in that** the said hole (O) and said edge (A₁, A₂) extend in a same plane at a tangent to said generating line.

3. Device according to claim 1,
**characterized in that** the cylindrical body (10) of the needle (7) comprises, in its outer cylindrical surface, at least one axial groove (16) ending at a predetermined distance from the tip (11) of the needle.

4. Device according to claim 3,
**characterized in that** the said groove (16) is of square or rectangular cross-section.

5. Device according to one of the claims 3 and 4,
**characterized in that** the said body (10) comprises four longitudinal grooves (16) diametrically opposed two by two.

6. Device according to one of the preceding claims,
**characterized in that** the cutting edge (A₁, A₂) has a pointed shape thanks to the grinding of two lateral faces (14, 15).

7. Device according to one of the preceding claims,
**characterized in that** the motion in translation of the needle (7) is controlled by an electric motor (M) supplied by a circuit including a detector (D) capable of detecting a parameter representative of the load moment generated when the tip of the needle (7) enters into contact with the stopper (2).
